# EUROPEAN PATENT SPECIFICATION

(11) **EP 2 647 694 B1**
(45) Date of publication and mention of the grant of the patent: **27.12.2017**
(21) Application number: 11838184.7
(22) Date of filing: 27.10.2011
(51) Int. Cl.: A23K 20/195

(54) **DEAD LACTOBACILLUS BIOMASS FOR ANTIMICROBIAL USE AND A PRODUCTION METHOD THEREFOR**
BIOMASSE AUS TOTEM LACTOBACILLUS ZUR VERWENDUNG ALS ANTIMIKROBIKUM UND HERSTELLUNGSVERFAHREN DAFÜR
BIOMASSE MORTE DE LACTOBACILLUS POUR UNE UTILISATION ANTIMICROBIENNE, ET SON PROCÉDÉ DE PRODUCTION

(30) Priority: 27.10.2011 KR 20110110381; 04.11.2010 KR 20100108971
(43) Date of publication of application: 09.10.2013
(73) Proprietor: Cell Biotech Co., Ltd., Gyeonggi-do 415-872 (KR); Chung, Myung-jun, Seocho-gu, Seoul 130-070 (KR)
(72) Inventor: CHUNG, Myung Jun, Seoul 137-070 (KR)
(74) Representative: Epping - Hermann - Fischer
(86) International application number: PCT/KR2011/008090
(87) International publication number: WO 2012/060579

(56) References cited:
- EP-A1- 1 308 506
- WO-A1-2005/087241
- KR-A- 20030 021 298
- KR-A- 20040 084 167
- KR-A- 20090 024 175
- KR-A- 20100 037 309
- F. CANDUCCI ET AL: "A lyophilized and inactivated culture of Lactobacillus acidophilus increases Helicobacter pylori eradication rates", ALIMENTARY PHARMACOLOGY & THERAPEUTICS, vol. 14, no. 12, 27 December 2000 (2000-12-27), pages 1625-1629, XP055110958, ISSN: 0269-2813, DOI: 10.1046/j.1365-2036.2000.00885.x
- SIMAKACHORN N ET AL: "Clinical evaluation of the addition of lyophilized, heat-killed Lactobacillus acidophilus LB to oral rehydration therapy in the treatment of acute diarrhea in children", JOURNAL OF PEDIATRIC GASTROENTEROLOGY AND NUTRITION, LIPPINCOTT WILLIAMS WILKINS, INC, US, vol. 30, no. 1, 1 January 2000 (2000-01-01), pages 68-72, XP009101369, ISSN: 0277-2116, DOI: 10.1097/00005176-200001000-00020
- BERNET-CAMARD M-F ET AL: "The human Lactobacillus acidophilus strain LA1 secretes a nonbacteriocin antibacterial substance(s) active in vitro and in vivo", APPLIED AND ENVIRONMENTAL MICROBIOLOGY, AMERICAN SOCIETY FOR MICROBIOLOGY, US, vol. 63, no. 7, 1 July 1997 (1997-07-01), pages 2747-2753, XP002231951, ISSN: 0099-2240
- S. STRASSER ET AL: "Influence of lyophilization, fluidized bed drying, addition of protectants, and storage on the viability of lactic acid bacteria", JOURNAL OF APPLIED MICROBIOLOGY, vol. 107, no. 1, 1 July 2009 (2009-07-01), pages 167-177, XP055026447, ISSN: 1364-5072, DOI: 10.1111/j.1365-2672.2009.04192.x
- ZARATE G ET AL: "Viability and biological properties of probiotic vaginal lactobacilli after lyophilization and refrigerated storage into gelatin capsules", PROCESS BIOCHEMISTRY, ELSEVIER, NL, vol. 41, no. 8, 1 August 2006 (2006-08-01) , pages 1779-1785, XP027984191, ISSN: 1359-5113 [retrieved on 2006-08-01]
- MORGAN C A ET AL: "Preservation of micro-organisms by drying; A review", JOURNAL OF MICROBIOLOGICAL METHODS, ELSEVIER, AMSTERDAM, NL, vol. 66, no. 2, 1 August 2006 (2006-08-01) , pages 183-193, XP027926942, ISSN: 0167-7012 [retrieved on 2006-08-01]

## Description

### Technical Field

The present invention relates to an antibacterial material comprising a tyndallized dead lactic acid bacteria lyophilizate and a dried tyndallized fermented filtrate, and more particularly, to an antibacterial material comprising a tyndallized dead lactic acid bacteria lyophilizate having a homogeneous appearance and an excellent antibacterial activity and prepared by performing an indirect heating process at an appropriate temperature and flow rate in a heat exchanger, a rapid cooling process for tyndallization, a fluidized-bed-drying process, and a freeze-drying process, and a method of preparing the antibacterial material.

### Background Art

In general, live lactic acid bacteria are settled in intestines and have various physiological activities to activate intestinal movement, to inhibit harmful bacteria, to reduce blood cholesterol, and to provide an anticancer effect. On the other hand, due to lactic acid and physiological active material (antibacterial active material) generally generated by lactic acid bacteria, tyndallized and freeze-dried bacteria lyophilizate (including dead bacteria lyophilizate) increase intestinal acidity and slow down peristaltic movement in the small intestine and to help digestive absorption, and control intestinal movement in the large intestine to prevent diarrhea.

That is, E. coli and Enterococcus that are intestinal bacteria from among intestinal flora are increased in intestines and thus intractable diarrhea is caused. A tyndallized and freeze-dried (antibacterial active) material of lactic acid bacteria and its fermented broth inhibits the growth of harmful bacteria and thus provides an improvement to diarrhea. Also, one of its essential functions is an immunity-boosting function. As a defense mechanism of the body, in order to heal and prevent diseases, a tyndallized and freeze-dried (dead) material of lactic acid bacteria activates microphages for sensing harmful bacteria in an immune system to rapidly sense bacteria and viruses, and generates gamma interferon to increase immunity and to cope with a disease.

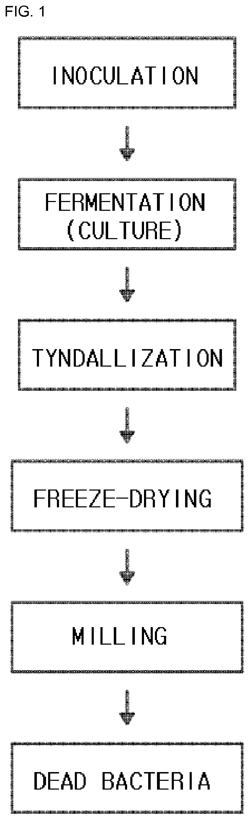

A conventional process of preparing a tyndallized and freeze-dried material of lactic acid bacteria is as illustrated in FIG. 1.

Tyndallization, freeze-drying, and milling processes are performed on a fermented broth of lactic acid bacteria. In this case, the tyndallization process is performed tyndallizing the fermented broth at 60 to 120°C for 10 to 60 minutes using a pressure container, and then rapidly cooling the fermented broth to a temperature equal to or less than 20°C. However, in the above-described conventional tyndallization method, due to carbonization during tyndallization, the appearance of dead lactic acid bacteria is very bad, the tyndallization is not appropriated performed, and thus a ratio of live lactic acid bacteria is excessively high.

In more detail, if the tyndallization temperature is increased, the completely prepared tyndallized and freeze-dried lactic acid bacteria has an excessively low titer, i.e., an excessively low bacteriostatic activity, their appearance is also excessively thick, and there are problems for commercialization. Otherwise, if the tyndallization temperature is lowered, since live lactic acid bacteria remain in the tyndallized and freeze-dried material, the tyndallized and freeze-dried lactic acid bacteria may not function properly as dead lactic acid bacteria and the live lactic acid bacteria may be misrecognized as contaminated bacteria.

Also, the number of live lactic acid bacteria differs according to a culture medium for fermenting lactic acid bacteria, and thus the number of dead lactic acid bacteria after a tyndallization process greatly differs. Therefore, a product may not easily have a homogeneous appearance, and bacteriostatic activity varies according to the number of dead lactic acid bacteria in a tyndallized and freeze-dried material.

EP 1308506 A1 discloses a mixture of bacteria suitable for the preservation of foods, which is a non starter culture, free from metabolites and comprises at least one first bacterium selected from the species Propionibackerium jensenii and at least one second bacterium selected from the genus Lactobacillus.

Canducci F. et al.: "A lyophilized and inactivated culture of Lactobacillus acidophilus increases Helicobacter pylori eradication rates2; Alimentary Pharmacology & Therapeutics, Vol. 14, No. 12 (2000-12-27), pages 1625-1629 and Simakachorn N. et al.: "Clinical evaluation of the addition of lyophilized, heat-killed Lactobacillus acidophilus LB to oral rehydration therapy in the treatment of acute diarrhea in children", Journal of Pediatric Gastroenterology and Nutrition, Lippincott Williams Wilkins, Inc, US, Vol. 30, No. 1 (2000-01-01), pages 68-72 both disclose that a lyophilized and inactivated culture of Lactobacillus acidophilus increases Heliobacter pylori eradication rates.

### Disclosure of Invention

### Technical Problem

The present invention provides method of preparing an antibacterial material according to claim 1. Preferred embodiments are set forth in the dependent claims.

### Technical Solution

According to the present invention, there is provided a method of preparing an antibacterial material comprising a tyndallized dead lactic acid bacteria lyophilizate and a dried tyndallized fermented filtrate, the method including the steps of fermenting lactic acid bacteria to form a fermented broth; tyndallizing the fermented broth; separating the tyndallized fermented broth into a tyndallized dead lactic acid bacteria-containing solution and a tyndallized fermented filtrate; freeze-drying the tyndallized dead lactic acid bacteria-containing solution so as to form a tyndallized dead lactic acid bacteria lyophilizate; fluidized-bed-drying or freeze-drying the tyndallized fermented filtrate so as to form a dried tyndallized fermented filtrate; and mixing the tyndallized dead lactic acid bacteria lyophilizate and the dried tyndallized fermented filtrate.

The tyndallizing of the fermented broth may include indirectly heating the fermented broth at 80 to 160°C at a flow rate of 10 to 100 ℓ/min. using a heat exchanger; and rapidly cooling the fermented broth to 20 to 40°C so as to kill the lactic acid bacteria.

The fermenting of the lactic acid bacteria may include fermenting the lactic acid bacteria after adding saccharide, yeast extract, soy peptone, an ionic component, and a growth factor.

The freeze-drying of the tyndallized dead lactic acid bacteria-containing solution and the tyndallized fermented filtrate may include rapidly freezing the tyndallized dead lactic acid bacteria-containing solution and the tyndallized fermented filtrate at -60 to -40°C; and drying the tyndallized dead lactic acid bacteria-containing solution and the tyndallized fermented filtrate at 20 to 60°C for 24 to 96 hours.

According to another aspect of the present invention, there is provided a method of preparing an antibacterial material comprising a tyndallized dead lactic acid bacteria lyophilizate and a dried tyndallized fermented filtrate, the method including the steps of fermenting lactic acid bacteria to form a fermented broth; separating the fermented broth into a lactic acid bacteria-containing solution and a fermented filtrate; tyndallizing the lactic acid bacteria-containing solution so as to form tyndallized dead lactic acid bacteria-containing solution; tyndallizing the fermented filtrate so as to form tyndallized fermented filtrate; freeze-drying the tyndallized dead lactic acid bacteria-containing solution so as to form tyndallized dead lactic acid bacteria lyophilizate; fluidized-bed-drying the tyndallized fermented filtrate so as to form a dried tyndallized fermented filtrate; and mixing the tyndallized dead lactic acid bacteria lyophilizate and the dried tyndallized fermented filtrate.

The configuration of the present invention will now be described in detail with respect to each step of the method.

### (a) Fermenting of lactic acid bacteria

In the step (a), lactic acid bacteria are fermented.

The fermenting of lactic acid bacteria may include adding saccharide, yeast extract, soy peptone, an ionic component, and a growth factor; and fermenting the lactic acid bacteria.

Also, the saccharide, the yeast extract, the soy peptone, and the ionic component are components required for survival and growth when the lactic acid bacteria are fermented, and the contents of the components may be 1 to 5 weight% of the saccharide, 0.1 to 5 weight% of the yeast extract, 0.1 to 5 weight% of the soy peptone, and 0.01 to 1 weight% of the ionic component with respect to a total weight of an aqueous protein solution. The saccharide may be glucose, lactose, or a mixture thereof. In addition, vitamin B-complex or TWEEN 80 may be further added as the growth factor.

The ionic component may be, but not limited to, ammonium citrate, sodium acetate, dipotassium phosphate, magnesium sulfate, manganese sulfate, or sodium chloride. The substances for culturing the lactic acid bacteria may be sterilized before the lactic acid bacteria are added.

### (b) Tyndallizing of the lactic acid bacteria

In the step (b), the lactic acid bacteria are tyndallized by heating the lactic acid bacteria to a high temperature and then cooling the lactic acid bacteria. According to the present invention, tyndallization may be performed before a fermented broth of lactic acid bacteria is separated into a lactic acid bacteria-containing solution and a fermented filtrate.

In this case, the tyndallization uses an indirect heating method using a heat exchanger. The appearance of tyndallized dead lactic acid bacteria may be improved to be homogeneous, a residual ratio of live lactic acid bacteria may be reduced to almost 0%, and an excellent bacteriostatic activity may be achieved by indirectly heating the lactic acid bacteria at 70 to 160°C at a flow rate of 1 to 200 ℓ/min., and rapidly cooling the lactic acid bacteria to 10 to 60°C. At the above temperature and the flow rate, improvement of the appearance of tyndallized dead lactic acid bacteria, a reduction in a residual ratio of live lactic acid bacteria, and an excellent bacteriostatic activity may be ensured.

In the present invention, "tyndallization" refers to an operation of killing lactic acid bacteria at a certain temperature to obtain dead lactic acid bacteria. Although live lactic acid bacteria are very weak to acid and thus may not easily reach intestines, dead lactic acid bacteria are hardly influenced by an environment of, for example, gastric acid and thus may reach small and large intestines, and the dead lactic acid bacteria and its dried fermented filtrate have antibacterial activities in the small and large intestines.

Alternatively, according to the present invention, tyndallization may be individually performed after a fermented broth of lactic acid bacteria is separated into a lactic acid bacteria-containing solution and a fermented filtrate. In this case, the tyndallization may be performed by heating the lactic acid bacteria-containing solution at 70 to 130°C using a steam autoclave, and rapidly cooling the lactic acid bacteria-containing solution at 10 to 60°C, and by heating the fermented filtrate at 70 to 130°C using a pressure container, and rapidly cooling the fermented filtrate to 10 to 60°C.

### (c) Separating into a lactic acid bacteria-containing solution and a fermented filtrate

In the step (c), the tyndallized fermented broth is separated into a tyndallized dead lactic acid bacteria-containing solution and a tyndallized fermented filtrate. The separating may be performed at 5,000 to 15,000 RPM using a centrifuge, and the tyndallized fermented broth is separated after centrifugation into a tyndallized dead lactic acid bacteria-containing solution settled to the bottom and a tyndallized fermented filtrate that is a supernatant.

### (d) Freeze-drying of the tyndallized dead lactic acid bacteria-containing solution

In the step (d), the tyndallized dead lactic acid bacteria-containing solution is coated with a cryoprotectant and then is freeze-dried so as to form tyndallized dead lactic acid bacteria lyophilizate. The cryoprotectant may be trehalose, maltodextrin, starch, or powdered skim milk, may be added by 5 to 40 weight% with respect to a total weight of the tyndallized dead lactic acid bacteria-containing solution, and may be added in the form of an aqueous solution. The freeze-drying may include rapidly freezing the tyndallized dead lactic acid bacteria-containing solution at -60 to -40°C; and drying the tyndallized dead lactic acid bacteria-containing solution at 20 to 60°C for 24 to 96 hours.

### (e) Drying of the tyndallized fermented filtrate

In the step (e), the tyndallized fermented filtrate separated in the step (c) is fluidized-bed-dried or freeze-dried so as to form a tyndallized and dried fermented filtrate. The fluidized-bed-drying of the tyndallized fermented filtrate may be performed by spraying the tyndallized fermented filtrate with an absorbent so as to dry the tyndallized fermented filtrate. In this case, the absorbent may be isolated soy protein, powdered skim milk, starch, or a mixture thereof, and may be added by 10 to 50 weight% with respect to a total weight of the tyndallized fermented filtrate.

The freeze-drying may be performed by 2X to 10X concentrating the tyndallized fermented filtrate using an evaporator (a concentrator), adding a cryoprotectant and an absorbent, rapidly cooling the tyndallized fermented filtrate at -60 to -40°C, and then drying the tyndallized fermented filtrate at 20 to 60°C for 24 to 96 hours. In this case, each of the cryoprotectant and the absorbent may be added by 5 to 40 weight% with respect to a total weight of the tyndallized and concentrated fermented filtrate.

### (f) Mixing of the tyndallized dead bacteria lyophilizate and the tyndallized and dried fermented filtrate

In the step (f), the tyndallized dead bacteria lyophilizate and the tyndallized and dried fermented filtrate are mixed. The tyndallized dead bacteria lyophilizate and the tyndallized and dried fermented filtrate obtained in steps (d) and (e) are individually milled and then mixed, thereby preparing the ultimate antibacterial material.

In this case, a product including the tyndallized dead lactic acid bacteria lyophilizate may be diluted using an appropriate diluent to allow the tyndallized dead lactic acid bacteria to have a concentration of 1.0×10⁹ to 1.0×10¹¹ cfu/g. In the concentration range of the tyndallized dead lactic acid bacteria, an optimal antibacterial activity may be achieved and a homogeneous appearance may be ensured.

The tyndallized dead lactic acid bacteria lyophilizate obtained according to the method of the present invention has an excellent antibacterial activity, and thus may be added into and used in various medicines, foods, cosmetics, and feeds to inhibit the growth of bacteria. In particular, the tyndallized dead lactic acid bacteria lyophilizate may be used in a pharmacological composition, a food composition, and a feed composition.

According to a reference example, there is provided an antibacterial pharmacological composition including the tyndallized dead lactic acid bacteria lyophilizate.

The tyndallized dead lactic acid bacteria lyophilizate according to the reference example may be used in an antibacterial pharmacological composition. In this case, the tyndallized dead lactic acid bacteria lyophilizate may be added to have a concentration of 1.0×10⁹ to 1.0×10¹¹ cfu/g in the composition.

Also, the tyndallized dead lactic acid bacteria lyophilizate according to the reference example may be used in a composition for intestinal regulation. In this context, "intestinal regulation" refers to treatment and improvement of disorders caused by abnormal fermentation of intestinal flora of animals. The disorders include, for example, infectious diarrhea, gastroenteritis, inflammatory bowel disease, neurogenic bowel syndrome, bacterial overgrowth in small intestines, intestinal acute diarrhea, and the like due to harmful microorganisms.

The composition may be prepared and administered in various dosage forms and using various methods as well known in the art. For example, the lactic acid bacteria or their culture according to the present invention may be mixed with a carrier generally used in the pharmaceutical field and may be prepared and administered in the form of tablets, troches, capsules, an elixir, a syrup, powder, a suspension, granules, or the like.

The carrier may be, but not limited to, for example, a binder, a glidant, a disintegrant, an excipient, a solubilizing agent, a dispersing agent, a stabilizer, a suspending agent, a coloring, or a flavoring. For example, the powder may be prepared by merely mixing the tyndallized dead lactic acid bacteria lyophilizate with a pharmaceutically allowed appropriate excipient such as lactose, starch, or amorphous cellulose.

The granules may be prepared by mixing the tyndallized dead lactic acid bacteria lyophilizate with a pharmaceutically allowed appropriate excipient, and a pharmaceutically allowed appropriate binder such as polyvinylpyrrolidone or hydroxypropyl cellulose, and then performing a wet granulation method using a solvent such as water, ethanol, or isopropanol, or a dry granulation method using a compression force. Also, the tablets may be prepared by mixing the granules with a pharmaceutically allowed appropriate lubricant such as magnesium stearate, and then forming tablets using a tablet machine.

The composition may be administered using an oral method. Also, a dose may be appropriately selected according to absorptivity, inactivity, or excretion speed of an active component in the body and age, sex, condition, or disease level of a patient. For example, 100 to 5000 mg with respect to a total weight of the tyndallized dead lactic acid bacteria lyophilizate composition may be administered once to three times a day for one person.

According to another reference example, there is provided an antibacterial food composition including the tyndallized dead lactic acid bacteria lyophilizate.

The tyndallized dead lactic acid bacteria lyophilizate may be used in an antibacterial food composition. In this case, the tyndallized dead lactic acid bacteria lyophilizate may be added to have a concentration of 1.0×10⁹ to 1.0×10¹¹ cfu/g in the composition.

Types of foods to which the tyndallized dead lactic acid bacteria lyophilizate composition is applicable are not particularly restricted. For example, the foods include drinks, teas, breads, chocolates, candies, snacks, and vitamin complexes, and include all functional foods. Also, the composition may be prepared as a health functional food by additionally adding a sitologically allowable food additive and forming as a medicine.

The health functional food may additionally include various materials such as a flavoring agent, natural carbohydrate, a sweetening agent, a vitamin, an electrolyte, a coloring agent, pectic acid, alginic acid, organic acid, a protective colloidal thickener, a pH regulator, a stabilizer, a preservative, glycerin, alcohol, and a carbonating agent.

According to another reference example, there is provided an antibacterial feed composition including the tyndallized dead lactic acid bacteria lyophilizate.

The tyndallized dead lactic acid bacteria lyophilizate may be used in an antibacterial feed composition. In this case, the tyndallized dead lactic acid bacteria lyophilizate may be added to have a concentration of 1.0×10⁹ to 1.0×10¹¹ cfu/g in the composition.

The feed composition may be used as a substitute for a conventional antibiotic, may improve a weight gain and a meat texture of livestock and may increase a milk yield and a level of immunity by inhibiting the growth of harmful bacteria in intestines, stably maintaining intestinal flora, and making a good health state of livestock. The feed composition may be prepared as, but not limited to, a fermented feed, an assorted feed, pellets, or a silage.

The fermented feed may be prepared by adding the tyndallized dead lactic acid bacteria lyophilizate and various microorganisms or enzymes and fermenting an organic material, and the assorted feed may be prepared by mixing various general feeds and the tyndallized dead lactic acid bacteria lyophilizate. Also, the pellet-type feed may be prepared by applying heat and pressure to the fermented feed or the assorted feed in a pellet machine.

### Brief Description of Drawings

FIG. 1 is a flow chart of a conventional method of preparing tyndallized dead lactic acid bacteria lyophilizate.
FIGS. 2 through 5 are flow charts of methods of preparing the antibacterial material including the tyndallized dead lactic acid bacteria lyophilizate, according to embodiments of the present invention.

### Best Mode for carrying out the Invention

Hereinafter, the present invention will be described in detail by explaining embodiments of the invention with reference to the attached drawings. The following embodiments are provided to gain a sufficient understanding of the present invention and not to limit the scope of the present invention. Matters not provided in the following descriptions can be sufficiently and technically inferred by one of ordinary skill in the art and thus will not be described here.

Test examples for comparing the antibacterial material including the tyndallized dead lactic acid bacteria lyophilizate prepared according to the present invention to those prepared according to a conventional method will now be described.

### [Comparative Example 1]

As illustrated in FIG. 1, a conventional method of preparing the tyndallized dead lactic acid bacteria lyophilizate was used. Specifically, a completely fermented broth of lactic acid bacteria was tyndallized at 60 to 120°C for 10 to 60 minutes using a pressure container such as a fermentation tube and then was rapidly cooled to a temperature equal to or less than 20°C. After that, the fermented broth was freeze-drying and milled, thereby obtaining the dead lactic acid bacteria lyophilizate. Results of the above test are as shown in Tables 1 and 2.

Table 1 shows the number and a residual ratio of live lactic acid bacteria (cells/g) according to a tyndallization temperature and a tyndallization time.

**[Table 1]**

| tyndallization temperature (°C)/ tyndallization time (min.) | 10 min. | 30 min. | 60 min. |
|---|---|---|---|
| 60 | 1.7E+09 | 6.9E+07 | 7.0E+06 |
| 80 | 3.9E+06 | 8.1E+04 | 4.4E+03 |
| 100 | 7.9E+03 | 6.2E+02 | 8.3E+01 |
| 120 | 1.1E+02 | Trace | Trace |

As shown in Table 1, in the tyndallization of lactic acid bacteria using a pressure container such as a fermentation tube, a residual ratio of live lactic acid bacteria according to the tyndallization temperature and the tyndallization time may be checked, and the tyndallization temperature has to be equal to or greater than 120°C in consideration of the residual ratio of live lactic acid bacteria. (The number of live lactic acid bacteria before the tyndallization was 8.6E+09.)

Table 2 shows a turbidity of dead lactic acid bacteria according to a tyndallization temperature and a tyndallization time.

**[Table 2]**

| tyndallization temperature (°C)/ tyndallization time (min.) | 10 min. | 30 min. | 60 min. |
|---|---|---|---|
| 60 | 0.030 | 0.032 | 0.030 |
| 80 | 0.032 | 0.035 | 0.047 |
| 100 | 0.038 | 0.052 | 0.074 |
| 120 | 0.062 | 0.136 | 0.281 |

As shown in Table 2, in the tyndallization of lactic acid bacteria using a pressure container such as a fermentation tube, the appearance (turbidity) of tyndallized dead lactic acid bacteria after the tyndallization depends on the tyndallization temperature and the tyndallization time, and the turbidity is high when the tyndallization temperature is equal to or greater than 100°C and the tyndallization time is equal to or greater than 30 minutes. If the turbidity is excessively high, commercialization and marketing is not easy. If the tyndallization temperature is excessively high, bacteriostatic activity will be reduced due to a bad appearance and a low antibacterial activity. Otherwise, if the tyndallization temperature is excessively low, a residual ratio of live lactic acid bacteria will be increased. Likewise, if the tyndallization time is excessively long, due to carbonization, dead lactic acid bacteria will have a bad appearance and a low bacteriostatic activity.
<1-1> Product Appearance: 0.1g of the product was dissolved with 10mℓ of purified water, and its turbidity was measured (OD610nm, Spectrophotometer).
<1-2> Number of Live Bacteria: The number of live lactic acid bacteria (cells/g) in the product after tyndallization was counted.

### [Embodiment 1]

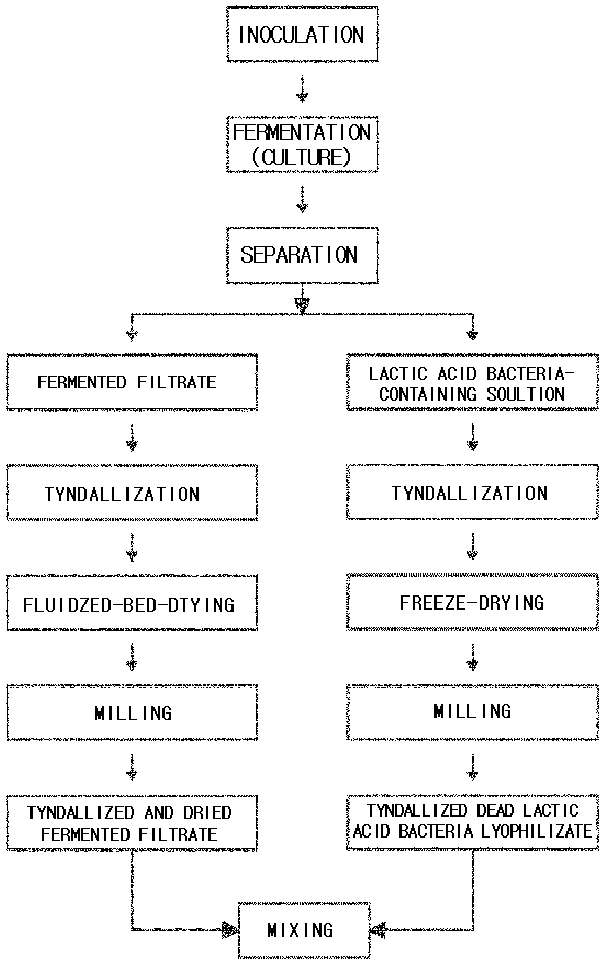

As illustrated in FIG. 5, a method of preparing tyndallized dead lactic acid bacteria lyophilizate, according to the present invention, was used.

Specifically, a completely fermented broth was separated into a fermented filtrate and a lactic acid bacteria-containing solution using a centrifuge of 7,260 RPM. The separated filtrate was transferred to a pressure container (a fermentation tank), was tyndallized at 90°C for 10 minutes, was cooled to 40°C, was re-tyndallized at 110°C for 10 minutes, was cooled to 40°C, and was 2X concentrated using a fluidized bed dryer, and isolated soy protein and powdered skim milk as an absorbent, thereby preparing a tyndallized and dried fermented filtrate.

The lactic acid bacteria-containing solution was uniformly mixed and diluted with purified water at 1:2, was tyndallized in a steam autoclave at 121°C for 20 minutes, was coated with 20 weight% of trehalose and starch as a cryoprotectant with respect to the tyndallized dead lactic acid bacteria-containing solution at room temperature, and then was pre-frozen and freeze-dried, thereby obtaining tyndallized dead lactic acid bacteria lyophilizate. The obtained tyndallized and dried fermented filtrate and the tyndallized dead lactic acid bacteria lyophilizate were individually milled and mixed.

### [Embodiment 2]

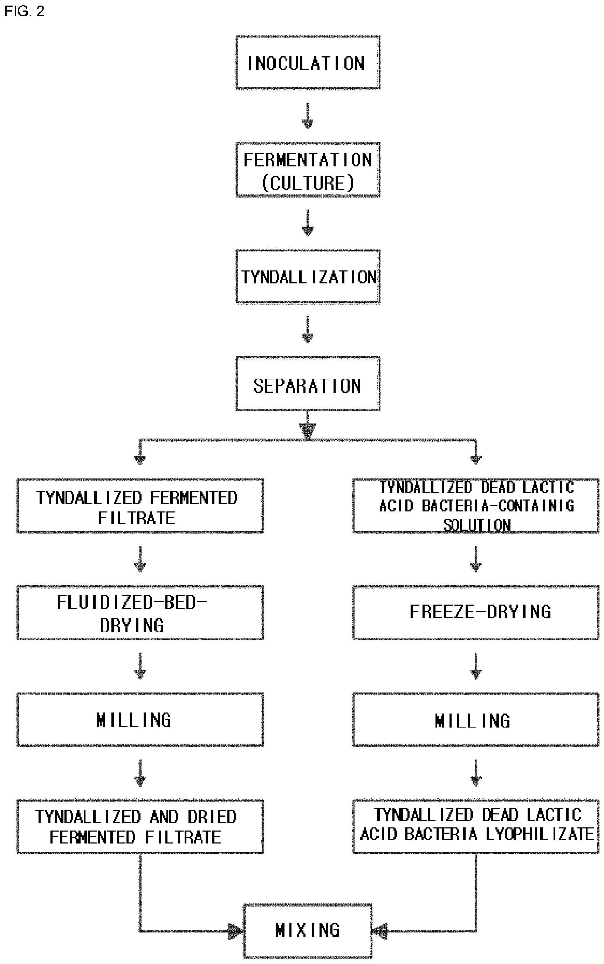

As illustrated in FIG. 2, a method of preparing tyndallized dead lactic acid bacteria lyophilizate, according to the present invention, was used.

A completely fermented broth of lactic acid bacteria was tyndallized at 90°C for 10 minutes, was cooled to 40°C, was re-tyndallized at 110°C for 10 minutes, was cooled to 40°C, and was separated into a tyndallized dead lactic acid bacteria-containing solution and a tyndallized fermented filtrate using a centrifuge. Then, the tyndallized fermented filtrate was 2X concentrated using a fluidized bed dryer, and isolated soy protein and powdered skim milk as an absorbent, thereby preparing tyndallized and dried fermented filtrate.

The tyndallized dead lactic acid bacteria-containing solution was coated with 20 weight% of trehalose and starch as a cryoprotectant with respect to the tyndallized dead lactic acid bacteria-containing solution at room temperature, and then was pre-frozen and freeze-dried, thereby obtaining tyndallized dead lactic acid bacteria lyophilizate. After that, the tyndallized and dried fermented filtrate and the tyndallized dead lactic acid bacteria lyophilizate were individually milled and mixed, thereby completing the process.

### [Embodiment 3]

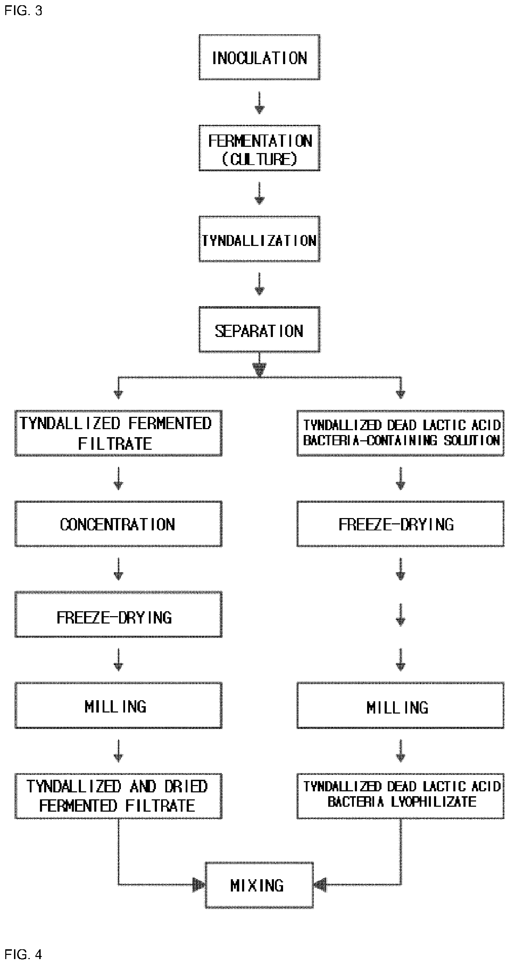

As illustrated in FIG. 3, a method of preparing tyndallized dead lactic acid bacteria lyophilizate, according to the present invention, was used.

A completely fermented broth of lactic acid bacteria was tyndallized at 90°C for 10 minutes, was cooled to 40°C, was re-tyndallized at 110°C for 10 minutes, was cooled to 40°C, and was separated into a tyndallized dead lactic acid bacteria-containing solution and a tyndallized fermented filtrate using a centrifuge. Then, the separated tyndallized fermented filtrate was 5X concentrated using an evaporator (a concentrator), was frozen in a freezer for 48 hours at a temperature equal to or less than -40°C using 20 weight% of isolated soy protein and powdered skim milk as an absorbent with respect to the tyndallized fermented filtrate, was freeze-dried in a freeze-dryer for 96 hours, and was milled, thereby obtaining tyndallized and dried fermented filtrate.

The tyndallized dead lactic acid bacteria-containing solution was coated with 20 weight% of trehalose and starch as a cryoprotectant with respect to the tyndallized dead lactic acid bacteria-containing solution at room temperature, and then was pre-frozen and freeze-dried, thereby obtaining tyndallized dead lactic acid bacteria lyophilizate. After that, the tyndallized and dried fermented filtrate and the tyndallized dead lactic acid bacteria lyophilizate were individually milled and mixed and an appropriate diluent was added, thereby completing the process.

### [Embodiment 4]

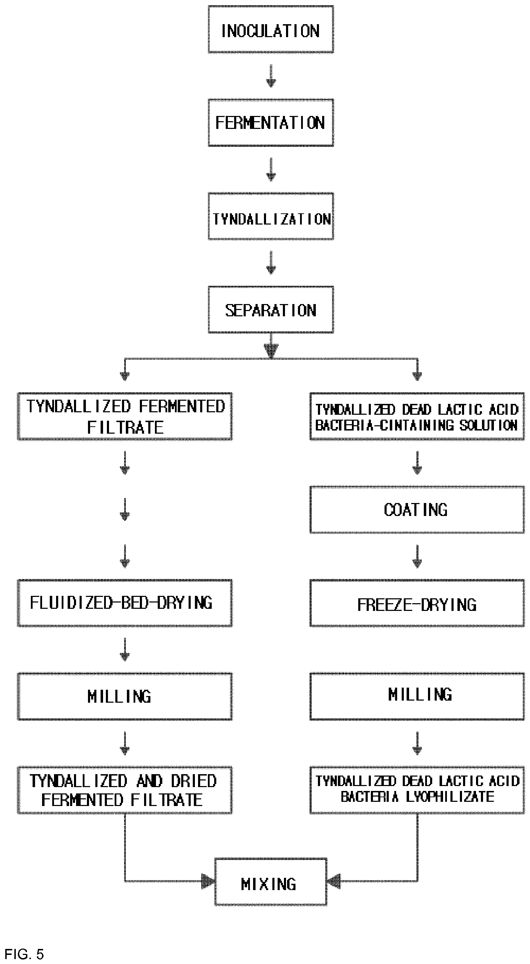

As illustrated in FIG. 4, a method of preparing tyndallized dead lactic acid bacteria lyophilizate, according to the present invention, was used.

A completely fermented broth of lactic acid bacteria was tyndallized in a heat exchanger system (UHT, manufactured by α-Laval) at 125°C at a flow rate of 30 ℓ/min. and was cooled to a temperature equal to or less than 40°C. Then, the completely cooled tyndallized fermented broth was separated into a tyndallized dead lactic acid bacteria-containing solution and a tyndallized fermented filtrate including an antibacterial active material, using a centrifuge (SC-35 Separator, manufactured by Westfalia).

The tyndallized fermented filtrate including the antibacterial active material was sprayed and dried in a fluidized bed dryer including 50 weight% of isolated soy protein and powdered skim milk as an absorbent with respect to the fermented filtrate, thereby forming a tyndallized and dried fermented filtrate, and the separated tyndallized dead lactic acid bacteria-containing solution was coated with 20 weight% of trehalose and starch as a cryoprotectant with respect to the tyndallized dead lactic acid bacteria-containing solution, and then was dried in a freeze-dryer at 40°C for 96 hours, thereby obtaining tyndallized dead lactic acid bacteria lyophilizate. After that, the tyndallized and dried fermented filtrate and the tyndallized dead lactic acid bacteria lyophilizate were individually milled and then were mixed in a drum mixer.

Test examples for measuring antibacterial activity in Embodiments 1 to 4 will now be described.

### [Test Example 1]

With regard to the antibacterial material including the tyndallized dead lactic acid bacteria lyophilizate prepared in Embodiments 1 to 4, the appearance and the number of live lactic acid bacteria (a residual ratio) were measured, and antibacterial activity was checked by performing a bacteriostatic test.

In this case, Salmonella paratyphi, Shigella genus, and Escherichia genus were used as bacteria, and the bacteriostatic test was performed using a bacteriostatic test method disclosed in "References and Test Methods for Medicines etc., 2^{nd} Revision" issued by the Korea Food and Drug Administration.

The test method is described below.
<1-1> Product Appearance: 0.1g of the product was dissolved with 10mℓ of purified water, and its turbidity was measured (OD610nm, Spectrophotometer).
<1-2> Number of Live Bacteria: The number of live lactic acid bacteria (cells/g) in the product after tyndallization was counted.
<1-3> Bacteriostatic Test
   - Test Bacteria and Test Bacteria Solution: Salmonella paratyphi A was used as test bacteria. The test bacteria were cultured in casein peptone (Tryptic Soy Agar) at 37°C for 18 to 24 hours, and then the number of bacteria (cells/g) was counted.
   - Preparation of Test Solution A: Accurately 1.7g of each of the antibacterial material prepared using the above tyndallization methods was put into 20mℓ of a liquid medium for suspending test bacteria, was stirred at room temperature for 5 to 10 minutes, and was centrifugally separated at 5,000 RPM for 15 minutes. Then, a supernatant was taken and was filtered and sterilized.
   - Preparation of Test Solution B: Accurately 2.4g of each of the antibacterial material prepared using the above tyndallization methods was manipulated and was prepared as in the test solution A.
   - Manipulation Method: 5mℓ of the test solution A, the test solution B, and a comparative medium were individually put into three sterilized test tubes having an inside diameter of 16mm and a length of 160mm, and were inoculated with accurately 0.1mℓ of the test bacteria solution (Salmonella paratyphi). 5mℓ of the test solution A and the test solution B were individually put into two additional test tube and were used as blank test solutions. These test tubes were cultured at 37°C and the growths of bacteria were observed with reference to the individual blank test tubes.
   - Determination: After the culturing process for 5 to 6 hours, bacteria should not be grown in the test solution A and the test solution B and should be grown in the comparative medium. After 24 hours, bacteria should be actively grown in the comparative medium and should not be grown in the test solution B.

Results of the above test are as shown in Table 3.

**[Table 3]**

| Tyndallization Method | Appearance | Number of Live Bacteria | | Bacteriostatic Activity | | | |
|---|---|---|---|---|---|---|---|
| | | | | 5 Hours | | 24 Hours | |
| | | After Tyndallization | In Product | A | B | A | B |
| Embodiment 1 | 0.096 | >E+05 | >E+05 | 1.21E+07 | 1.30E+07 | 5.70E+08 | 8.52E+07 |
| Embodiment 2 | 0.130 | 5.82E+03 | 6.51E+04 | 1.20E+07 | 1.17E+07 | 2.50E+09 | 8.40E+07 |
| Embodiment 3 | 0.147 | 1.28E+03 | 5.94E+04 | 1.19E+07 | 1.13E+07 | 8.19E+09 | 6.00E+08 |
| Embodiment 4 | 0.031 | Trace | <E+02 | 1.12E+07 | 9.93E+06 | 5.82E+06 | <E+06 |

As shown in Table 3, in comparison to the tyndallized dead lactic acid bacteria lyophilizate of Embodiments 1 to 3, the tyndallized dead lactic acid bacteria lyophilizate of Embodiment 4, which are prepared using the preparing method according to the present invention, have a lower turbidity and thus have a homogeneous appearance, have a much less number of live lactic acid bacteria, and have the best bacteriostatic activity. However, in comparison to Comparative Example 1, the appearance is homogeneous and the bacteriostatic activity is excellent in Embodiments 1 to 3.

### [Test Example 2]

In tyndallization of a fermented broth using a heat exchanger, according to the temperature of a heating part that receives heat of the heat exchanger, a retention time (a flow rate) in a tube (a holding tube) in which tyndallization actually occurs, and the temperature of a cooling part, the quality of tyndallization may differ and a titer (bacteriostatic activity) may also differ. Accordingly, the appearance, the number of live lactic acid bacteria, and bacteriostatic activity of the tyndallized dead lactic acid bacteria lyophilizate according to each process condition were checked.

### <2-1> Test of the appearance and the number of live bacteria of lactic acid bacteria after tyndallization according to a flow rate when a heating temperature was 140°C and a cooling temperature was 40°C in a heat exchanger

The appearances and the numbers of live bacteria of lactic acid bacteria after tyndallization according to a flow rate when the temperature of a heating part that receives heat of a heat exchanger was 140°C, and the temperature of a cooling part was 40°C were compared.

**[Table 4]**

| Flow Rate (ℓ/min.) | Appearance | Number of Live Bacteria after Tyndallization |
|---|---|---|
| 1 | 0.120 | Trace |
| 40 | 0.032 | Trace |
| 60 | 0.030 | < E+02 |
| 120 | 0.028 | > E+05 |

As shown in Table 4, if the flow rate is excessively low, a retention time in the heat exchanger is long, over sterilization occurs, and thus the appearance is inappropriate. If the flow rate is excessively high, the retention time is short, tyndallization (sterilization) does not appropriately occur, and thus the number of live lactic acid bacteria after tyndallization is large.

### <2-2> Test of the appearance and the number of live bacteria of lactic acid bacteria after tyndallization according to a heating temperature when a flow rate was 40 ℓ/min. and a cooling temperature was 40°C in a heat exchanger

The appearances and the numbers of live bacteria of lactic acid bacteria after tyndallization according to the temperature of a heating part when a retention time in a tube, i.e., a flow rate, was 40 ℓ/min. and the temperature of a cooling part was 40°C were compared.

**[Table 5]**

| Heating Temperature (°C) | Appearance | Number of Live Bacteria after Tyndallization |
|---|---|---|
| 50 | 0.027 | > E+05 |
| 70 | 0.028 | > E+05 |
| 140 | 0.031 | Trace |
| 170 | 0.058 | Trace |

As shown in Table 5, if the heating temperature of the heat exchanger is excessively low, tyndallization (sterilization) does not occur appropriately and thus the number of live bacteria after tyndallization is large. If the heating temperature is excessively high, due to the temperature, carbonization partially occurs and thus the appearance is in appropriate.

### <2-3> Test of the appearance and the number of live bacteria of lactic acid bacteria after tyndallization according to a cooling temperature when a heating temperature was 140°C and a flow rate was 40 ℓ/min. in a heat exchanger

The appearances and the numbers of live bacteria of lactic acid bacteria after tyndallization according to the temperature of a cooling part when the temperature of a heating temperature that receives heat of a heat exchanger was 140°C and a flow rate was 40 ℓ/min. were compared.

**[Table 6]**

| Cooling Temperature (°C) | Appearance | Number of Live Bacteria after Tyndallization |
|---|---|---|
| 10 | 0.031 | Trace |
| 40 | 0.030 | Trace |
| 60 | 0.030 | Trace |
| 80 | 0.032 | <E+02 |

As shown in Table 6, if the cooling temperature is equal to or lower than 60°C, there is no significant difference in appearance and the number of live bacteria after tyndallization, only the consumption of a utility is increased, and thus an optimal condition is determined as about 60°C.

### <2-4> Measurement of the appearance, the number of live bacteria, and bacteriostatic activity according to the temperature of a heating part when a flow rate is 120 ℓ/min.

The appearance, the number of live bacteria, and bacteriostatic activity according to the temperature of a heating part that receives heat of a heat exchanger when a retention time in a tube, i.e., a flow rate, was 120 ℓ/min. were measured (Table 7). The measuring method of Test Example 1 was used, and the temperature of a cooling part was set as 40°C.

**[Table 7]**

| Temperature of Heating Part (°C) | Appearance | Number of Live Bacteria | | Bacteriostatic Activity | | | |
|---|---|---|---|---|---|---|---|
| | | | | 5 Hours | | 24 Hours | |
| | | After Tyndallization | In Product | A | B | A | B |
| 60 | 0.028 | >E+05 | >E+05 | 1.33E+07 | 1.23E+07 | 5.70E+09 | 1.52E+09 |
| 90 | 0.030 | 4.7E+03 | 1.1E+04 | 2.01 E+07 | 1.13E+07 | 1.77E+09 | 9.97E+08 |
| 150 | 0.034 | <E+03 | <E+03 | 1.57E+07 | 1.19E+07 | 1.08E+07 | 6.54E+06 |

### <2-5> Measurement of the appearance, the number of live bacteria, and bacteriostatic activity according to the temperature of a heating part when a flow rate was 30 ℓ/min.

The appearance, the number of live bacteria, and bacteriostatic activity according to the temperature of a heating part that receives heat of a heat exchanger when a retention time in a tube, i.e., a flow rate, was 30 ℓ/min. were measured (Table 8). The measuring method of Test Example 1 was used, and the temperature of a cooling part was set as 40°C.

**[Table 8]**

| Temperature of Heating Part (°C) | Appearance | Number of Live Bacteria | | Bacteriostatic Activity | | | |
|---|---|---|---|---|---|---|---|
| | | | | 5 Hours | | 24 Hours | |
| | | After Tyndallization | In Product | A | B | A | B |
| 90 | 0.035 | >E+03 | >E+04 | 1.70E+07 | 9.80E+06 | 8.70E+08 | 2.22E+08 |
| 120 | 0.038 | Trace | <E+02 | 1.63E+07 | 1.17E+07 | 4.30E+07 | 1.07E+07 |
| 150 | 0.031 | Trace | <E+02 | 3.19E+07 | 1.43E+07 | 9.83E+06 | <E+06 |

### <2-6> Measurement of the appearance, the number of live bacteria, and bacteriostatic activity of a product according to a flow rate when the temperature of a heating part was 130°C

The appearance, the number of live bacteria, and bacteriostatic activity of a product when the temperature of a heating part that receives heat of a heat exchanger was 130°C were measured (Table 9). The measuring method of Test Example 1 was used, and the temperature of a cooling part was set as 20°C.

**[Table 9]**

| Flow Rate (ℓ/min.) | Appearance | Number of Live Bacteria | | Bacteriostatic Activity | | | |
|---|---|---|---|---|---|---|---|
| | | | | 5 Hours | | 24 Hours | |
| | | After Tyndallization | In Product | A | B | A | B |
| 5 | 0.043 | Trace | Trace | 2.04E+07 | 9.65E+06 | <E+06 | <E+06 |
| 50 | 0.034 | Trace | <E+02 | 1.72E+07 | 1.47E+07 | 9.73E+06 | 4.65E+06 |
| 100 | 0.032 | <E+02 | 2.09E+02 | 9.89E+06 | 1.63E+07 | 3.30E+07 | 2.06E+07 |

### <2-7> Measurement of the appearance, the number of live bacteria, and bacteriostatic activity of a product according to a flow rate when the temperature of a heating part was 150°C

The appearance, the number of live bacteria, and bacteriostatic activity of a product when the temperature of a heating part that receives heat of a heat exchanger was 150°C were measured (Table 10). The measuring method of Test Example 1 was used, and the temperature of a cooling part was set as 40°C.

**[Table 10]**

| Flow Rate (ℓ/min.) | Appearance | Number of Live Bacteria | | Bacteriostatic Activity | | | |
|---|---|---|---|---|---|---|---|
| | | | | 5 Hours | | 24 Hours | |
| | | After Tyndallization | In Product | A | B | A | B |
| 5 | 0.032 | Trace | Trace | 1.01E+07 | 1.66E+07 | <E+05 | <E+05 |
| 50 | 0.030 | Trace | <E+02 | 1.63E+07 | 9.88E+06 | 3.02E+06 | <E+06 |
| 100 | 0.032 | Trace | <E+02 | 2.16E+07 | 1.69E+07 | 2.87E+07 | 4.26E+06 |

### <2-8> Measurement of the appearance, the number of live bacteria, and bacteriostatic activity of a product according to a cooling temperature when the temperature of a heating part was 140°C and a flow rate was 35 ℓ/min.

The appearance, the number of live bacteria, and bacteriostatic activity of a product when the temperature of a heating part that receives heat of a heat exchanger was 125°C and a flow rate was 35 ℓ/min. were measured (Table 11). The measuring method of Test Example 1 was used.

**[Table 11]**

| Cooling Temperature (°C) | Appearance | Number of Live bacteria | | Bacteriostatic Activity | | | |
|---|---|---|---|---|---|---|---|
| | | | | 5 Hours | | 24 Hours | |
| | | After Tyndallization | In Product | A | B | A | B |
| 20 | 0.036 | Trace | <E+02 | 1.44E+07 | 1.33E+07 | <E+06 | <E+06 |
| 40 | 0.033 | Trace | <E+02 | 1.78E+07 | 2.68E+07 | <E+06 | <E+06 |
| 50 | 0.035 | Trace | <E+02 | 1.47E+07 | 1.06E+07 | 1.53E+06 | <E+06 |

### <2-9> Measurement of the appearance, the number of live bacteria, and bacteriostatic activity according to the number of dead bacteria after mixing when the temperature of a heating part was 130°C, a flow rate was 35 ℓ/min., and a cooling temperature was 40°C

the appearance, the number of live bacteria, and bacteriostatic activity of a product when the temperature of a heating part that receives heat of a heat exchanger was 130°C, a flow rate was 35 ℓ/min., and a cooling temperature was 40°C were measured (Table 12). The measuring method of Test Example 1 was used.

**[Table 12]**

| Number of Dead Bacteria after Mixing (cells/g) | Appearance | Number of Live Bacteria | | Bacteriostatic Activity | | | |
|---|---|---|---|---|---|---|---|
| | | | | 5 Hours | | 24 Hours | |
| | | After Tyndallization | In Product | A | B | A | B |
| < 5.0E+09 | 0.032 | Trace | <E+02 | 1.34E+07 | 1.39E+07 | 3.78E+07 | 3.09E+07 |
| 3.0E+10 | 0.032 | Trace | <E+02 | 1.77E+07 | 1.88E+07 | 1.03E+06 | <E+06 |
| > 1.0E+11 | 0.033 | Trace | <E+02 | 1.64E+07 | 1.22E+07 | 1.19E+06 | <E+06 |

### Industrial Applicability

A tyndallized dead lactic acid bacteria lyophilizate obtained according to method of the present invention may have a homogeneous appearance and an improved antibacterial activity.

Also, in a method of preparing the antibacterial material comprising the tyndallized dead lactic acid bacteria lyophilizate and a dried tyndallized fermented filtrate, according to the present invention, since a fermented broth of lactic acid bacteria is separated into a lactic acid bacteria-containing solution and a fermented filtrate before or after a tyndallization process and the lactic acid bacteria-containing solution and the fermented filtrate are individually dried, the number of dead lactic acid bacteria may be artificially and variously adjusted and thus various products having a homogeneous appearance may be produced.

Furthermore, a method of preparing the antibacterial material comprising the tyndallized dead lactic acid bacteria lyophilizate and a dried tyndallized fermented filtrate, according to the present invention, may achieve process simplification and quality improvement using a heat exchanger for indirect and instantaneous tyndallization after lactic acid bacteria are fermented and before the fermented lactic acid bacteria are separated into bacteria and a fermented filtrate.

Accordingly, the antibacterial material and the preparing method thereof, according to the present invention, may be appropriately used to prepare various medicines, foods, feeds, cosmetics, etc. which require antibacterial activity and intestinal regulation.

## Claims

1. A method of preparing an antibacterial material comprising a tyndallized dead lactic acid bacteria lyophilizate and a dried tyndallized fermented filtrate, the method comprising the steps of:
fermenting lactic acid bacteria to form a fermented broth;
tyndallizing the fermented broth;
separating the tyndallized fermented broth into a tyndallized lactic acid bacteria-containing solution and a tyndallized fermented filtrate;
freeze-drying the tyndallized dead lactic acid bacteria-containing solution so as to form a tyndallized dead lactic acid bacteria lyophilizate;
fluidized-bed-drying or freeze-drying the tyndallized fermented filtrate so as to form a dried tyndallized fermented filtrate; and
mixing the tyndallized dead lactic acid bacteria lyophilizate and the dried tyndallized fermented filtrate, wherein the tyndallizing of the fermented broth comprises: indirectly heating the fermented broth at 80 to 160 °C at a flow rate of 10 to 100 ℓ/min using a heat exchanger; and rapidly cooling the fermented broth to 10 to 60 °C so as to kill the lactic acid bacteria.

2. The method of claim 1, wherein the fermenting of the lactic acid bacteria comprises fermenting the lactic acid bacteria after adding saccharide, yeast extract, soy peptone, and an ionic component.

3. The method of claim 1, wherein the freeze-drying of the tyndallized lactic acid bacteria-containing solution and the tyndallized fermented filtrate comprises:
rapidly freezing the tyndallized dead lactic acid bacteria-containing solution and the tyndallized fermented filtrate at -60 to -40°C; and
drying the tyndallized dead lactic acid bacteria-containing solution and the tyndallized fermented filtrate at 20 to 60°C for 24 to 96 hours.

## Patentansprüche

1. Verfahren zur Herstellung eines antibakteriellen Materials, umfassend ein tyndalisiertes Lyophilisat von toten Milchsäurebakterien und ein getrocknetes tyndalisiertes, fermentiertes Filtrat, wobei das Verfahren die folgenden Schritte umfasst:
Fermentieren der Milchsäurebakterien, um eine fermentierte Brühe zu bilden,
Tyndalisieren der fermentierten Brühe,
Auftrennen der tyndalisierten, fermentierten Brühe in eine tyndalisierte, Milchsäurebakterien enthaltende Lösung und ein tyndalisiertes, fermentiertes Filtrat,
Gefriertrocknen der tyndalisierten, tote Milchsäurebakterien enthaltenden Lösung, um ein tyndalisiertes Lyophilisat von toten Milchsäurebakterien zu bilden,
Fließbetttrocknen oder Gefriertrocknen des tyndalisierten, fermentierten Filtrats, um ein getrocknetes tyndalisiertes, fermentiertes Filtrat zu bilden, und
Mischen des tyndalisierten Lyophilisats von toten Milchsäurebakterien und des getrockneten tyndalisierten, fermentierten Filtrats, wobei das Tyndalisieren der fermentierten Brühe umfasst: indirektes Erwärmen der fermentierten Brühe bei 80 bis 160 °C bei einer Flussrate von 10 bis 100 l/min unter Verwendung eines Wärmetauschers und schnelles Abkühlen der fermentierten Brühe auf 10 bis 60 °C, um die Milchsäurebakterien abzutöten.

2. Verfahren nach Anspruch 1, wobei das Fermentieren der Milchsäurebakterien das Fermentieren der Milchsäurebakterien nach dem Zusetzen von Saccharid, Hefeextrakt, Sojapepton und einer ionischen Komponente umfasst.

3. Verfahren nach Anspruch 1, wobei das Gefriertrocknen der tyndalisierten, Milchsäurebakterien enthaltenden Lösung und des tyndalisierten, fermentierten Filtrats Folgendes umfasst:
schnelles Gefrieren der tyndalisierten, tote Milchsäurebakterien enthaltenden Lösung und des tyndalisierten, fermentierten Filtrats bei -60 bis -40 °C, und
Trocknen der tyndalisierten, tote Milchsäurebakterien enthaltenden Lösung und des tyndalisierten, fermentierten Filtrats über 24 bis 96 Stunden bei 20 bis 60 °C.

## Revendications

1. Procédé de préparation d'une matière antibactérienne comprenant un lyophilisat de bactéries d'acide lactique mortes tyndallisée et un filtrat fermenté tyndallisé séché, le procédé comprenant les étapes pour :
faire fermenter des bactéries d'acide lactique et former un bouillon fermenté ;
tyndalliser le bouillon fermenté ;
séparer le bouillon fermenté tyndallisé en une solution contenant des bactéries d'acide lactique tyndallisée et un filtrat fermenté tyndallisé :
lyophiliser la solution contenant des bactéries d'acide lactique mortes tyndallisée de manière à former un lyophilisat de bactéries d'acide lactique mortes tyndallisé ;
sécher en lit fluidisé ou lyophiliser le filtrat fermenté tyndallisé de manière à former un filtrat fermenté tyndallisé séché, et
mélanger le lyophilisat de bactéries d'acide lactique mortes tyndallisé et le filtrat fermenté tyndallisé séché, dans lequel la tyndallisation du bouillon fermenté comprend : le chauffage indirect du bouillon fermenté à 80 à 160 °C à un débit de 10 à 100 l/min à l'aide d'un échangeur de chaleur, et le refroidissement rapide du bouillon fermenté à 10 à 60 °C de manière à tuer les bactéries d'acide lactique.

2. Procédé selon la revendication 1, dans lequel la fermentation des bactéries d'acide lactique comprend la fermentation des bactéries d'acide lactique après ajout de saccharides, d'extrait de levure, de peptone de soja et d'un composant ionique.

3. Procédé selon la revendication 1, dans lequel la lyophilisation de la solution contenant des bactéries d'acide lactique tyndallisée et du filtrat fermenté tyndallisé comprend :
le séchage rapide de la solution contenant des bactéries d'acide lactique mortes tyndallisée et du filtrat fermenté tyndallisé à -60 °c à -40 °C, et
le séchage de la solution contenant des bactéries d'acide lactique mortes tyndallisée et du filtrat fermenté tyndallisé à 20 °C à 60 °C de 24 à 96 heures.
